# EUROPEAN PATENT APPLICATION

(11) **EP 4 485 148 A1**
(43) Date of publication of application: **01.01.2025**
(21) Application number: 24185807.5
(22) Date of filing: 01.07.2024
(51) Int. Cl.: G06F 3/01, A61B 5/0205, A61B 5/16, A61B 5/18

(54) **PUPIL DYNAMICS, PHYSIOLOGY, AND CONTEXT FOR ESTIMATING AFFECT AND WORKLOAD**

(30) Priority: 30.06.2023 US 202318216995
(71) Applicant: Rockwell Collins, Inc., Cedar Rapids, IA 52498 (US)
(72) Inventor: WU, Peggy, East Hartford, CT (US); RADLBECK, Andrew, East Hartford, CT (US); BLAKESLEE, Brigid A., East Hartford, CT (US); SUNDARAMOORTHI, Ganesh, East Hartford, CT (US)
(74) Representative: Dehns

(57) **Abstract**

A computer system records eye tracking data and identifies movements in the eye tracking data to determine gaze and pupil dynamics. The system also records physiological data such as heart rate, electroencephalogram (EEG), and functional near-infrared spectroscopy (fNIRs), and used the physiological data to determine an emotional state. Eye tracking data is correlated with a current task; the system determines a performance metric based on eye tracking data, and applies a positive or negative emotional weighting.

## Description

### BACKGROUND

Cognitive workload is difficult to measure. Cognitive workload is generally measured via self-report surveys. This process is time consuming, can take a trainee out of a simulation, affecting the realism of the experience, and can itself add to the cognitive workload. Furthermore, emotion (or affect) is known to influence cognitive workload, but is rarely taken into account during self-reports. Negative emotions can increase the self-report workload, which in turn inflates the workload measurement.

Consequently, it would be advantageous if an apparatus existed that is suitable for monitoring pilot workload in light of the pilot's emotional state.

### SUMMARY

In one aspect, there is provided a computer system that records eye tracking data. The system identifies movements in the eye tracking data to determine gaze and pupil dynamics. The system also records physiological data such as heart rate, electroencephalogram (EEG), and functional near-infrared spectroscopy (fNIRs), and used the physiological data to determine an emotional state. Eye tracking data is correlated with a current task; the system determines a performance metric based on eye tracking data, and applies a positive or negative emotional weighting.

It is to be understood that both the foregoing general description and the following detailed description are exemplary and explanatory only and should not restrict the scope of the invention which is defined by the claims. The accompanying drawings, which are incorporated in and constitute a part of the specification, illustrate exemplary embodiments of the inventive concepts disclosed herein and together with the general description, serve to explain the principles.

### BRIEF DESCRIPTION OF THE DRAWINGS

The numerous advantages of the embodiments of the inventive concepts disclosed herein may be better understood by those skilled in the art by reference to the accompanying figures in which:
FIG. 1 shows a block diagram of a system suitable for implementing embodiments of the incentive concepts disclosed herein;
FIG. 2 shows a flowchart of an exemplary embodiment of the inventive concepts disclosed herein;
FIG. 3 shows a block diagram of a neural network according an exemplary embodiment of the inventive concepts disclosed herein.

### DETAILED DESCRIPTION

Before explaining at least one embodiment of the inventive concepts disclosed herein in detail, it is to be understood that the inventive concepts are not limited in their application to the details of construction and the arrangement of the components or steps or methodologies set forth in the following description or illustrated in the drawings. In the following detailed description of embodiments of the instant inventive concepts, numerous specific details are set forth in order to provide a more thorough understanding of the inventive concepts. However, it will be apparent to one of ordinary skill in the art having the benefit of the instant disclosure that the inventive concepts disclosed herein may be practiced without these specific details. In other instances, well-known features may not be described in detail to avoid unnecessarily complicating the instant disclosure. The inventive concepts disclosed herein are capable of other embodiments or of being practiced or carried out in various ways within the scope of the claims. Also, it is to be understood that the phraseology and terminology employed herein is for the purpose of description and should not be regarded as limiting.

**As** used herein a letter following a reference numeral is intended to reference an embodiment of the feature or element that may be similar, but not necessarily identical, to a previously described element or feature bearing the same reference numeral (e.g., 1, 1a, 1b). Such shorthand notations are used for purposes of convenience only, and should not be construed to limit the inventive concepts disclosed herein in any way unless expressly stated to the contrary.

Further, unless expressly stated to the contrary, "or" refers to an inclusive or and not to an exclusive or. For example, a condition A or B is satisfied by anyone of the following: A is true (or present) and B is false (or not present), A is false (or not present) and B is true (or present), and both A and B are true (or present).

In addition, use of the "a" or "an" are employed to describe elements and components of embodiments of the instant inventive concepts. This is done merely for convenience and to give a general sense of the inventive concepts, and "a' and "an" are intended to include one or at least one and the singular also includes the plural unless it is obvious that it is meant otherwise.

Finally, as used herein any reference to "one embodiment," or "some embodiments" means that a particular element, feature, structure, or characteristic described in connection with the embodiment is included in at least one embodiment of the inventive concepts disclosed herein. The appearances of the phrase "in some embodiments" in various places in the specification are not necessarily all referring to the same embodiment, and embodiments of the inventive concepts disclosed may include one or more of the features expressly described or inherently present herein, or any combination of sub-combination of two or more such features, along with any other features which may not necessarily be expressly described or inherently present in the instant disclosure.

Broadly, embodiments of the inventive concepts disclosed herein are directed to a computer system that records eye tracking data. The system identifies movements in the eye tracking data to determine gaze and pupil dynamics. The system also records physiological data such as heart rate, electroencephalogram (EEG), and functional near-infrared spectroscopy (fNIRs), and used the physiological data to determine an emotional state. Eye tracking data is correlated with a current task; the system determines a performance metric based on eye tracking data, and applies a positive or negative emotional weighting.

This invention uses data of a pilot's eyes to detect their gaze and pupil dynamics, coupled with knowledge about their current task to differentiate between a pilot exhibiting task appropriate vigilance as opposed to dangerous attention tunneling or fixation. A task flow diagram represents the operator tasks. Interactions between the pilot and the instrumentation are used to estimate the probability distribution of the task the pilot is currently conducting, and physiological measures including electroencephalogram (EEG) and functional near-infrared spectroscopy (fNIRs) are used to determine neuroactivity to reduce the probability of false positives and increase the accuracy of the pilot vigilance monitoring system.

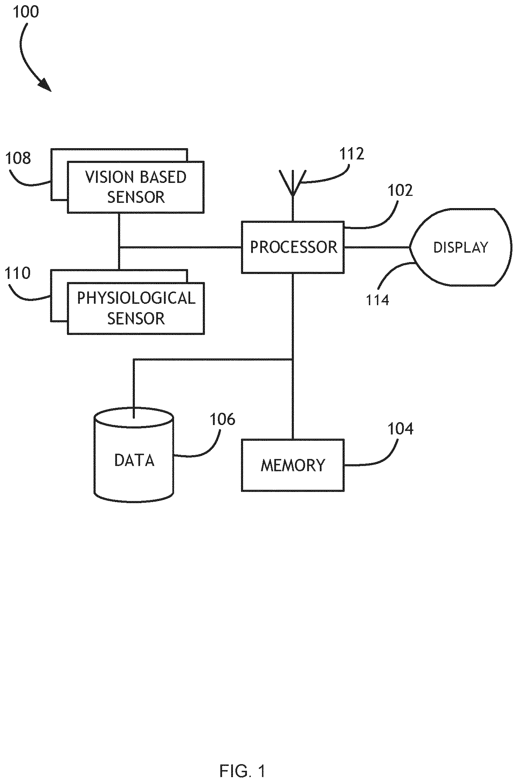

Referring to FIG. 1, a block diagram of a system 100 suitable for implementing embodiments of the incentive concepts disclosed herein is shown. The system 100 includes a processor 102, memory 104 in data communication with the processor 102 for storing processor executable code, one or more eye tracking sensors / cameras 108 for receiving eye tracking data stream, and one or more physiological sensors 110. Physiological sensors 110 may include devices such as a heart rate sensor, an electroencephalograph (EEG), functional near-infrared spectroscopy (fNIRs), or any other such biometric data sensing device.

In at least one embodiment, the eye tracking sensors 108 record eye movement / gaze of a pilot and eye lid position. The processor executable code configures the processor 102 to continuously log the eye tracking data in a data storage element 106. The processor 102 analyzes the eye tracking data to identify gaze and pupil dynamics (e.g., pupil response and changes overtime).

Eye tracking data are correlated with discreet portions of a training scenario or task, and / or specific stimuli such as instrument readings, alerts, or the like. The processor 102 characterizes the eye tracking data with respect to the scenario or task; for example, the processor 102 may retrieve scenario or task specific actions and / or metrics from a data storage device 106; the actions and / or metrics may include optimized instrument scan patterns and durations. The processor 102 may determine via the eye tracking data if the user conformed to the optimized instrument scan pattern and duration within some threshold. The degree of conformity may generally correspond to a measure of the user's workload. Alternatively, or in addition, the processor 102 may analyze the eye tracking data separate from any specific scenario or task to characterize the user's workload. It may be appreciated that scan patterns and pupil dynamics may be correlated with user workload.

In at least one embodiment, the processor 102 determines whether the eye tracking data indicates attention to certain instrumentation within bounds of acceptable attention. In some instances, specific limitations to the amount of time a user spends observing an instrument may indicate "fixation" or "attention tunneling". Attention tunneling may indicate user workload.

The processor 102 also receives physiological data from one or more physiological sensors 110. In at least one embodiment, the processor 102 correlates eye tracking data (including at least gaze and pupil dynamics) with the physiological data and determines an emotional state (positive or negative) based on the physiological data. The processor 102 weights the workload characterization according to the emotional state. For example, where a user exhibits a negative emotional state, the characterized workload may be greater than if the user exhibited a positive emotional state.

**The** processor 102 may compare the eye tracking and physiological data to stored profiles. Such profiles may be specific to the user, including weightings defined by emotional state; for examples, specific users may be more prone to emotion-based workload adjustment. Furthermore, the processor 102 may compare physiological data to stored profiles defining emotional states for the specific user with respect to the physiological data. For example, heightened heart rate and increased hemodynamic activity may be correlated with a positive emotional state in some users but a negative emotional state in others.

In at least one embodiment, the processor 102 may alert a remote party such as ground control personnel via a wireless communication device 112. Alternatively, or in addition, the processor 102 may render the determination of excessive workload on a display 114. For example, in a training scenario, an instructor may continuously monitor workload on the display 114.

In at least one embodiment, the processor 102 transfers the stored eye tracking data and other correlated system and task data to an offline storage device for later analysis and correlation to historic data and other outside factors such as crew rest, crew sleet rhythms, flight schedules, etc. Such transfer may be in real time via the wireless communication device 112.

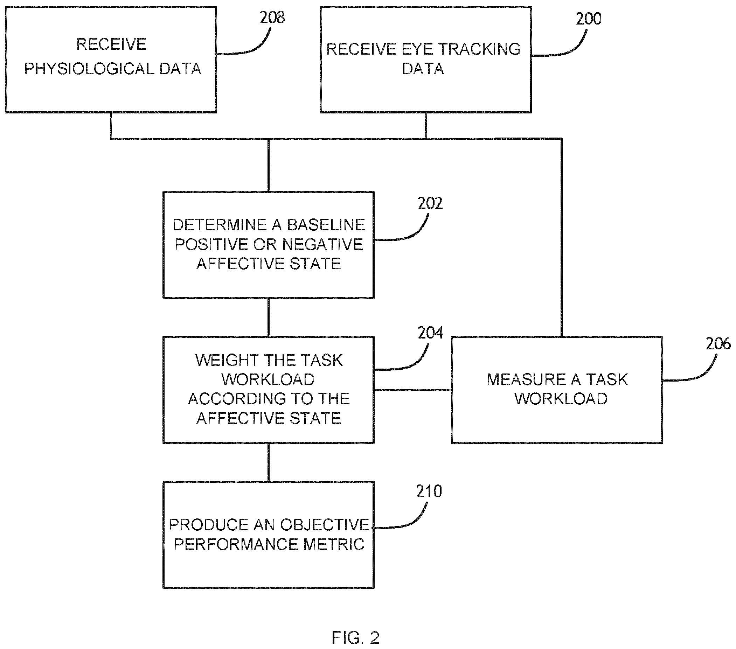

Referring to FIG. 2, a flowchart of an exemplary embodiment of the inventive concepts disclosed herein is shown. A computer system implementing embodiments of the inventive concepts disclosed herein receives 200 an image stream corresponding to eye tracking data from one or more vision-based sensors, and continuously logs the eye tracking data. Gaze, pupil dynamics, and eye lid position are used to determine 206 user workload, either absolutely or in relation to a training scenario or task. In at least one embodiment, determining 206 workload may also comprise receiving 208 physiological data. Such analysis may include processing via machine learning, neural network algorithms.

Scenarios and tasks may define specific scan patterns and gaze durations for certain instruments or locations. Such specific scan patterns and gaze durations may be further defined by other stimuli or inputs; for example, when observing a radar, the number of targets presented may impact the amount of time reasonably necessary to absorb the information presented.

In at least one embodiment, the physiological data is produced via one or more physiological sensors such as a heart rate monitor, an EEG, and / or an fNIRs. Such physiological data is used to determine 202 an emotional state of the user. Emotional states may be characterized as either "positive" or "negative", or defined along some multi-dimensional spectrum. Emotional states provide a weighting factor for the determined workload.

In at least one embodiment, the determined workload is weighted 204 according to the emotional state to further characterize the workload (a high measured workload during a negative emotional state is normalized to a lower absolute level). Alternatively, or in addition, the determined workload is weighted 204 according to the emotional state to predict the user's response (a given workload may be characterized as more stressful during a negative emotional state).

In at least one embodiment, the system may produce an objective performance metric for the user corresponding to the given scenario / task, and stimuli. Furthermore, the system may initiate remedial action where the weighted workload exceeds some threshold. For example, the system may render warning messages to the user or contact a third party. Alternatively, or in addition, where an automation system exists, the monitoring system may automatically increase levels of automation to reduce user workload.

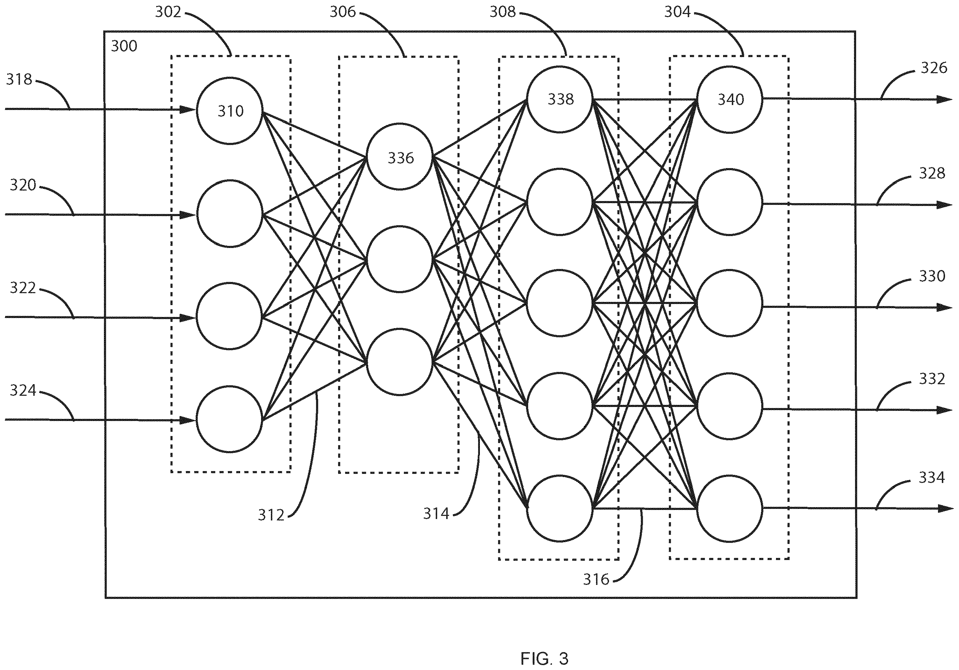

Referring to FIG. 3, a block diagram of a neural network 300 according an exemplary embodiment of the inventive concepts disclosed herein is shown. The neural network 300 comprises an input layer 302 that receives external inputs (including physiological signals, such as, heart rate, EEG, and fNIRs, eye tracking data, and potentially user or task specific profiles), and output layer 304, and a plurality of internal layers 306, 308. Each layer comprises a plurality of neurons or nodes 310, 336, 338, 340. In the input layer 302, each node 310 receives one or more inputs 318, 320, 322, 324 corresponding to a digital signal and produces an output 312 based on an activation function unique to each node 310 in the input layer 302. An activation function may be a Hyperbolic tangent function, a linear output function, and / or a logistic function, or some combination thereof, and different nodes 310, 336, 338, 340 may utilize different types of activation functions. In at least one embodiment, such activation function comprises the sum of each input multiplied by a synaptic weight. The output 312 may comprise a real value with a defined range or a Boolean value if the activation function surpasses a defined threshold. Such ranges and thresholds may be defined during a training process. Furthermore, the synaptic weights are determined during the training process.

Outputs 312 from each of the nodes 310 in the input layer 302 are passed to each node 336 in a first intermediate layer 306. The process continues through any number of intermediate layers 306, 308 with each intermediate layer node 336, 338 having a unique set of synaptic weights corresponding to each input 312, 314 from the previous intermediate layer 306, 308. It is envisioned that certain intermediate layer nodes 336, 338 may produce a real value with a range while other intermediated layer nodes 336, 338 may produce a Boolean value. Furthermore, it is envisioned that certain intermediate layer nodes 336, 338 may utilize a weighted input summation methodology while others utilize a weighted input product methodology. It is further envisioned that synaptic weight may correspond to bit shifting of the corresponding inputs 312, 314, 316.

An output layer 304 including one or more output nodes 340 receives the outputs 316 from each of the nodes 338 in the previous intermediate layer 308. Each output node 340 produces a final output 326, 328, 330, 332, 334 via processing the previous layer inputs 316, the final output 326, 328, 330, 332, 334 corresponding to a user emotional state, a characterization of workload with respect to the user emotional state, or both. Such outputs may comprise separate components of an interleaved input signal, bits for delivery to a register, or other digital output based on an input signal and DSP algorithm.

In at least one embodiment, each node 310, 336, 338, 340 in any layer 302, 306, 308, 304 may include a node weight to boost the output value of that node 310, 336, 338, 340 independent of the weighting applied to the output of that node 310, 336, 338, 340 in subsequent layers 304, 306, 308. It may be appreciated that certain synaptic weights may be zero to effectively isolate a node 310, 336, 338, 340 from an input 312, 314, 316, from one or more nodes 310, 336, 338 in a previous layer, or an initial input 318, 320, 322, 324.

In at least one embodiment, the number of processing layers 302, 304, 306, 308 may be constrained at a design phase based on a desired data throughput rate. Furthermore, multiple processors and multiple processing threads may facilitate simultaneous calculations of nodes 310, 336, 338, 340 within each processing layers 302, 304, 306, 308.

Layers 302, 304, 306, 308 may be organized in a feed forward architecture where nodes 310, 336, 338, 340 only receive inputs from the previous layer 302, 304, 306 and deliver outputs only to the immediately subsequent layer 304, 306, 308, or a recurrent architecture, or some combination thereof.

Embodiments of the inventive concepts disclosed herein are critical to enabling reduced crew or single pilot operations, and will provide independent measures necessary to facilitate reduced crew in the cockpit by providing a means to identify when crew members are unable to continue safe flight and notify relief crew or activate automation. Furthermore, a training application may utilize embodiments of the inventive concepts to compare the small involuntary eye movements of a pilot-in-training to previously characterized professional pilot data patterns and emotional states. Improved methods of assessing an operator's ability to acquire and maintain situation awareness is important for training. As such, it can serve as enabling technologies for single pilot operations or reduced crew operations.

It is believed that the inventive concepts disclosed herein and many of their attendant advantages will be understood by the foregoing description of embodiments of the inventive concepts disclosed, and it will be apparent that various changes may be made in the form, construction, and arrangement of the components thereof without departing from the broad scope of the inventive concepts disclosed herein or without sacrificing all of their material advantages; and individual features from various embodiments may be combined to arrive at other embodiments. The form herein before described being merely an explanatory embodiment thereof, it is the intention of the following claims to encompass and include such changes. Furthermore, any of the features disclosed in relation to any of the individual embodiments may be incorporated into any other embodiment.

## Claims

1. A computer apparatus comprising:
at least one eye tracking camera (108);
one or more physiological data recording devices (110); and
at least one processor (102) in data communication with a memory (104) storing processor executable code, wherein the processor executable code configures the at least one processor to:
receive an image stream from the at least one eye tracking camera;
identify gaze, pupil dynamics, and eye lid position from the image stream;
determine a workload metric based on the gaze, pupil dynamics, and eye lid position;
receive physiological data from the one or more physiological data recording devices;
determine an emotional state based on the physiological data; and
weight the determined workload metric according to the emotional state.

2. The computer apparatus of Claim 1, wherein determining the workload metric is further based on the physiological data.

3. The computer apparatus of Claim 2, wherein:
the processor executable code further configures the at least one processor to receive a task or user specific profile of gaze, pupil dynamics, and physiological data; and
determining the workload metric includes reference to the task or user specific profile.

4. The computer apparatus of Claim 3, wherein determining the emotional state includes reference to the task or user specific profile.

5. The computer apparatus of any preceding Claim, wherein at least one of the one or more physiological data recording devices comprises a functional near-infrared spectroscope, fNIRs.

6. The computer apparatus of any preceding Claim, wherein the processor executable code further configures the at least one processor to execute a remedial action if the weighted workload metric exceeds some threshold.

7. The computer apparatus of any preceding Claim, wherein the at least one processor embodies a trained neural network.

8. A method comprising:
receiving an image stream from at least one eye tracking camera (108);
identifying gaze, pupil dynamics, and eye lid position from the image stream;
determining a workload metric based on the gaze, pupil dynamics, and eye lid position;
receiving physiological data from one or more physiological data recording devices;
determining an emotional state based on the physiological data; and
weighting the determined workload metric according to the emotional state.

9. The method of Claim 8, wherein determining the workload metric is further based on the physiological data.

10. The method of Claim 9, further comprising receiving a task or user specific profile of gaze, pupil dynamics, and physiological data, wherein determining the workload metric includes reference to the task or user specific profile.

11. The method of Claim 10, wherein determining the emotional state includes reference to the task or user specific profile.

12. The method of Claim 11, wherein weighting the determined workload metric comprises predicting a user response to the workload metric based on the emotional state and task or user specific profile.

13. The method of any of Claims 8 to 12, further comprising executing a remedial action if the weighted workload metric exceeds some threshold.

14. A pilot monitoring system comprising a computer apparatus as claimed in any of claims 1 to 7.
